# EUROPÄISCHE PATENTANMELDUNG

(11) **EP 1 258 233 A1**
(43) Veröffentlichungstag der Anmeldung: **20.11.2002**
(21) Anmeldenummer: 01810500.7
(22) Anmeldetag: 18.05.2001
(51) Int. Cl.: A61F 2/46

(54) **Probierkugeln für Hüftgelenkprothesen**

(71) Anmelder: Sulzer Orthopedics Ltd., 6340 Baar (CH)
(72) Erfinder: Koller, Hansjörg, 8400 Winterthur (CH)
(74) Vertreter: Manitz, Finsterwald & Partner Gbr

(57) **Zusammenfassung**

Die Erfindung handelt von Probierkugeln für Hüftgelenkprothesen, welche eine Konusverbindung zwischen einem Prothesenhals (24) mit Konus (3) und aufsetzbaren Hüftgelenkkugeln aufweisen, wobei die Hüftgelenkkugeln und die Probierkugeln (1) jeweils praktisch gleiche Radien (21) und jeweils die gleiche Lage ihrer konischen Gegenfläche (4) zum Kugelmittelpunkt (11) aufweisen. Die Probierkugeln besitzen einen separaten Einsatz (2) mit vorbestimmter Federkraft für den Kontakt zum Konus (3), wobei der Einsatz (2) in den Probierkugeln (1) in unterschiedlichem Abstand (27) längs der Polachse (13) zum Kugelmittelpunkt (11) befestigbar ist.

## Beschreibung

Die Erfindung handelt von Probierkugeln für Hüftgelenkprothesen, welche eine Konusverbindung zwischen einem Prothesenhals mit Konus und aufsetzbaren Hüftgelenkkugeln aufweisen, wobei die Hüftgelenkkugeln und die zugehörigen Probierkugeln jeweils praktisch gleiche Radien und jeweils die gleiche Lage ihrer konischen Gegenfläche relativ zum Kugelmittelpunkt aufweisen.

Eine verbreitete Operationstechnik bei künstlichen Hüftgelenken sieht vor, dass nach dem Einsetzen eines Prothesenschaftes mit Konusverbindung für eine künstliche Hüftgelenkkugel zunächst Probierkugeln, die mit der Lage ihrer konischen Gegenfläche zum Kugelmittelpunkt den künstlichen Hüftgelenkkugeln entsprechen, vorgesehen werden, um die Spannung der Bänder im Gelenk bei Artikulation zu überprüfen. Dabei kann die Probierkugel einen geringfügig kleineren Durchmesser aufweisen, beispielsweise eine Reduktion um 0,3 mm, damit sie leichter in einer Lagerschale des Acetabulums einfahrbar ist. Der Arzt kann auf diese Weise mit leicht vom Schaft entfernbaren Kugeln, die in der Regel aus Kunststoff sind, um die Kugelschale nicht zu beschädigen, die Kugel mit der optimalen Eindringtiefe für den Konus finden und diese durch die endgültige Hüftgelenkkugel, welche mit einem festen Press-Sitz auf dem Konus verankert wird, ersetzen.

Eine analoge Operationstechnik sieht vor, die Probierkugeln auf einer eingeschlagenen Knochenraspel, welche die Prothesenform und einen gleichen Konus besitzt, aufzustecken um die Artikulation zu überprüfen. Der Konus der Raspel hat dann häufig eine zusätzliche Rille oder einen Ausschnitt, um dort die Kräfte beim Ausschlagen der Raspel auf die Raspel zu übertragen.

Eine bekannte Massnahme besteht darin in der konischen Gegenfläche einen Einstich anzubringen und dort einen O-Ring anzubringen, der über die konische Fläche vorsteht. Dieser O-Ring sollte trotz mehrfachem Verpressen und Sterilisieren seine Form und Elastizität behalten.

Nach dem Aufsetzen der Probierkugeln kommt es daher immer wieder vor, dass sich die konischen Flächen - beispielsweise durch Streifen an einem anderen Gegenstand - voneinander lösen. Dem soll die Erfindung entgegenwirken. Sie hat die Aufgabe eine unabsichtliches Abgleiten der Probierkugeln vom Konus zu verhindern. Diese Aufgabe wird dadurch erfüllt, dass die Probierkugeln einen separaten Einsatz mit vorbestimmter Federwirkung für den Kontakt zum Konus aufweisen, wobei der Einsatz in den Probierkugeln in unterschiedlichem Abstand längs der Polachse zum Kugelmittelpunkt befestigbar ist.

Die Erfindung hat den Vorteil, dass die Haltekraft für eine Probierkugel nicht durch die üblichen Massabweichungen wie Herstelltoleranzen und Langzeitgebrauch beeinflusst wird. Auch bei einer Verschiebung um Zehntel Millimeter in der Längsachse bleibt die Haltekraft praktisch unverändert.

Ein weiterer Vorteil besteht darin, dass die Probierkugel aus einem eher weichen Kunststoff bestehen kann, während der Einsatz aus einem hochbelastbaren Werkstoff bestehen kann.

Die abhängigen Ansprüche 2 bis 12 stellen vorteilhafte Weiterbildungen der Erfindung dar.

Die Erfindung hat den weiteren Vorteil, dass für eine Konusgrösse ein Standard-Einsatz verwendet werden kann, der in Probierkugeln mit unterschiedlichem Kugeldurchmesser und in Probierkugeln mit gleichem Kugeldurchmesser und unterschiedlicher Lage der konischen Gegenfläche zum Kugelmittelpunkt einsetzbar ist.

Der Einsatz kann aus einem spritzbaren Kunststoff in einem Spritzwerkzeug hergestellt werden, um über eine grosse Anzahl gleiche Abmessungen zu erhalten. Er kann aber auch wegen der Zugänglichkeit für Bearbeitungswerkzeuge aus Metallstangen auf einem Stangenautomat gefertigt werden.

Wenn der Einsatz die Form eines Bechers hat, dessen Rand durch Längsschlitze in Stützlappen und Biegefedern unterteilt ist, und die Biegefedern mit über die konische Gegenform vorstehenden Innen-Nocken versehen sind, wird der Konus beim Einsetzen durch die Stützlappen geführt und durch die Innen-Nocken der Biegefedern radial verpresst. Unter dem Einfluss dieser radialen Anpresskräfte verhindert die Reibung an den Innen-Nocken ein unabsichtliches Abgleiten vom Konus. Die Stützlappen können sich zusätzlich radial in der Probierkugel abstützen, um Überbelastungen bei verkantetem Einfahren des Konus zu verhindern.

Wenn auf der Innenseite der Probierkugel Längsnuten angebracht sind, können diese mit entsprechenden Aussen-Nocken der Biegefedern als Drehsicherung für den Einsatz verwendet werden. Sie können aber auch als Aufnahme beim Einspannen der halbfertigen Probierkugel für das Herstellen der Kugeloberfläche dienen. Mit einer Schnappverbindung, bei welcher der Einsatz leicht zusammengepresst und die Probierkugel leicht aufgeweitet wird können beide Teile zusammengefügt werden. Die Lage der Innen-Nocken am Einsatz kann in Längsrichtung so vorgesehen werden, dass für die gleiche Konusgrösse auch Rillen oder Ausnehmungen an den Konen von Raspeln durch die Nocken hintergriffen werden, um ein nicht gewolltes Abgleiten zu verhindern.

Bei den Konen der Schaftprothesen ist die Oberfläche häufig mit einem Feinstgewinde versehen, dessen Spitzen sich geringfügig verformen können, um bei harten Prothesenkugeln aus Metall oder aus Keramik lokale Spannungsspitzen zu verhindern. Solche Feinstgewinde vergrössern gleichzeitig die Reibung zwischen Innen-Nocken und Konus und verhindern ein Abgleiten vom Konus. Dadurch, dass die Radialkräfte der Biegefedern beherrschbar vorbestimmt sind, wird der Verschleiss an den Innen-Nocken klein gehalten. Im Gegensatz zu einer Probierkugel mit konischem Press-Sitz kann die Verbindung zur konischen Gegenfläche nicht überbelastet werden, da der Tiefenanschlag für den Konus an dessen Stirnfläche vorgesehen ist.

Im folgenden wird die Erfindung an Ausführungsbeispielen beschrieben.

Es zeigen:
- Fig. 1: Schematisch einen vergrösserten Längsschnitt durch eine erfindungsgemässe Probierkugel;
- Fig. 2: schematisch einen vergrösserten Längsschnitt durch die Probierkugel von Fig. 1, die auf den Konus einer Schaftprothese aufgesetzt ist;
- Fig. 3: schematisch einen vergrösserten Längsschnitt durch die Probierkugel von Fig. 1, die auf den Konus einer Raspel aufgesetzt ist;
- Fig. 4: schematisch und stark vergrössert einen Längsschnitt durch eine Probierkugel gemäss Fig. 2 beim Aufsetzen auf einen Konus;
- Fig. 5: schematisch und vergrössert eine Ansicht von unten der Probierkugel von Fig. 1, bei welcher der Einsatz noch nicht montiert wurde;
- Fig. 6: schematisch und vergrössert eine allgemeine Ansicht des Einsatzes von Fig. 1 vor seiner Befestigung in der Probierkugel; und
- Fig. 7: schematisch und vergrössert eine Probierkugel mit einem aus der Probierkugel vorstehenden Einsatz.

Die Figuren zeigen Probierkugeln für Hüftgelenkprothesen, welche eine Konusverbindung zwischen einem Prothesenhals 24 mit Konus 3 und aufsetzbaren Hüftgelenkkugeln aufweisen, wobei die Hüftgelenkkugeln und die Probierkugeln 1 jeweils praktisch gleiche Radien 21 und jeweils die gleiche Lage ihrer konischen Gegenfläche 4 zum Kugelmittelpunkt 11 aufweisen. Die Probierkugeln besitzen einen separaten Einsatz 2 mit vorbestimmter Federkraft für den Kontakt zum Konus 3, wobei der Einsatz 2 in den Probierkugeln 1 in unterschiedlichem Abstand 27 längs der Polachse 13 zum Kugelmittelpunkt 11 befestigbar ist.

In den Figuren sind gleiche Hinweiszeichen für gleiche Funktionen verwendet.

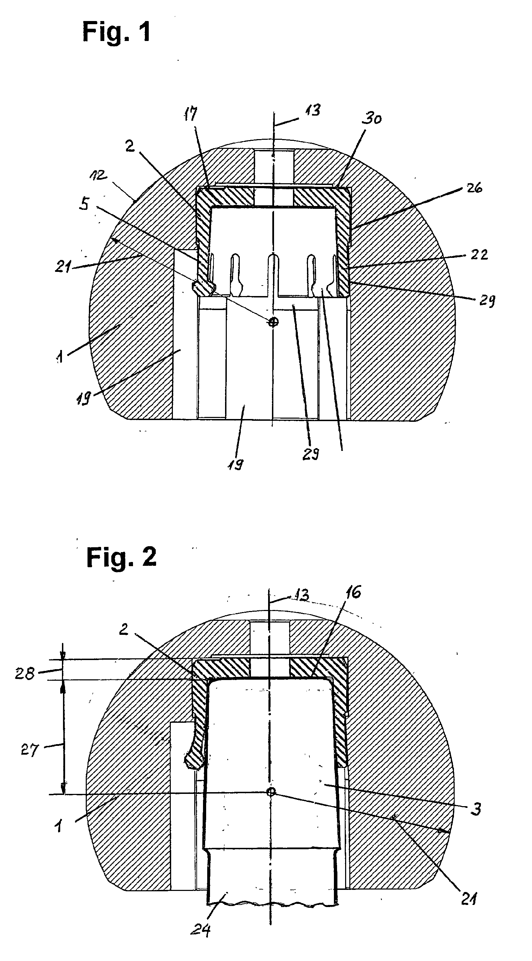

Die Probierkugel in den Figuren 1, 2, 3, 4 und 5 besteht aus einem eigentlichen Kugelkörper 1 aus Kunststoff, beispielsweise aus POM (Polyoximethylen), welches sich spanend gut bearbeiten lässt und andere Gegenstände nicht verletzt. Von der Kugeloberfläche 12 besteht noch mehr als die Hälfte. Längs ihrer Polachse 13 ist die Kugel von unten her aufgebohrt und in dieser Bohrung ist ein Einsatz 2 eingesetzt und mit einer Schnappverbindung 26 gehalten. Der Einsatz liegt mit seiner Stirnfläche 30 in der Kugel 1 auf und hat einen konischen Übergang zu einem radial vorstehenden, zylindrischen Vorsprung 33 der in einem Rücksprung der Bohrung gefangen ist. Der Einsatz 2 selbst hat die Form eines Bechers mit einer konischen Gegenfläche 4, welche für die Aufnahme von einem Konus 3 bestimmt ist und welche so bemessen ist, dass der Konus 3 vor dem Aufweiten der konischen Gegenfläche 4 mit seiner Stirnfläche am Bechergrund anschlägt. Die konische Gegenfläche 4 hat somit nur noch eine Funktion als Führung und radiale Abstützung. Bis in die halbe Tiefe des Bechers sind Längsschlitze 10 eingelassen, die jeweils Stützlappen 22 und Biegefedern 5 in umlaufender Richtung trennen. Entsprechend der Unterteilung in Stützlappen 22 und in Biegefedern 5 ist die Kugel 1 auf der Innenseite bearbeitet. Für die Stützlappen 22, welche die konische Gegenfläche 4 zum Becherrand verlängern um dem Konus 3 eine längere Führung zu geben sind konische Teilflächen 29 in der Kugel angebracht, welche ihrerseits als Stützflächen 20 die Stützlappen 22 stützen, wenn ein Konus 3 verkantet eingefahren wird. Die Erzeugung der konischen Teilflächen 29 wird dadurch erreicht, dass in der Bohrung zunächst eine konische Fläche ausgedreht wird, welche zur Basis in einen Zylinder 31 übergeht.

Anschliessend werden radiale Längsnuten 19 in einer Breite angebracht, dass die Biegefedern 5 in radialer Richtung in diese Nuten zurückfedern können. Die Biegefedern 5 besitzen zum Becherrand hin Innen-Nocken 6 und Aussen-Nocken 9. Die Aussen-Nocken 9 stehen soweit nach aussen vor, dass sie schon im unbelasteten Zustand der Biegefedern 5 in den Längsnuten 19 liegen und eine Drehsicherung 8 für den Einsatz 2 bilden.

Die eigentliche Haltekraft wird durch das Vorstehen der Innen-Nocken und die Stärke der Biegefedern bestimmt, die nur in ihrem elastischen Bereich belastet werden dürfen. Beim Einfahren eines Konus muss die Biegefeder um das Mass vom Vorsprung des Innen-Nockens 6 in die Längsnute 19 zurückfedern. Eine Einlauffläche 7 sorgt dafür, dass der Innen-Nocken 6 langsam nach aussen gepresst wird. Entsprechend dem Federweg der Biegefeder 5 ergibt sich eine Anpresskraft auf den Konus 3, die vorherbestimmt ist und die soviel Reibung zwischen Innen-Nocken 6 und Konus 3 erzeugt, dass ein Abgleiten vom Konus 3 verhindert wird. Bei symmetrischer Verteilung der Biegefedern 5 heben sich die Anpresskräfte am Konus 3 weitgehend auf und haben zentrierende Wirkung, während sich die durch die Anpresskräfte erzeugten Reibungskräfte addieren, um ein Abgleiten vom Konus 3 zu verhindern. Eine derartig erzeugte Haltekraft lässt sich nicht nur bei Probierkugeln mit einem Konus sondern auch bei Probierkugeln mit einem zylindrischen Sitz zu einem Schaft anwenden.

In die Stirnfläche 30 vom Einsatz 2 sind sternförmige Kanäle eingelassen, die eine Belüftung bis in die eigentliche Schnappverbindung 26 erlauben und Sicherheit beim Sterilisieren geben. Einsatz 2 und Probierkugel 1 weisen im Polbereich eine Bohrung 23 auf, durch welche die Stirnfläche 16 eines eingefahrenen Konus 3 kontrollierbar ist. Der Pol der Probierkugel 1 kann eine abgeplattete Fläche oder Vertiefung 34 aufweisen, in welcher eine Beschriftung zu ihrer Identifikation angebracht ist.

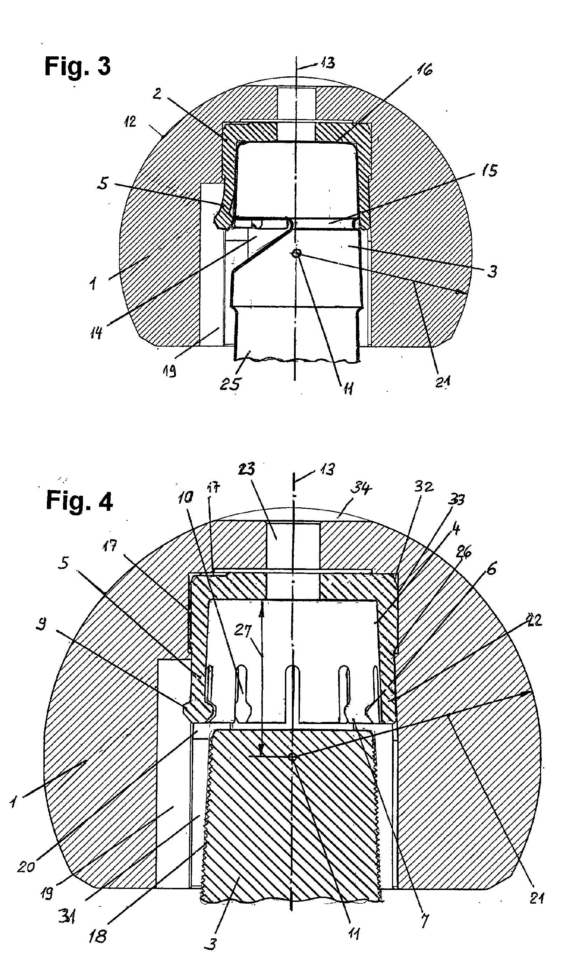

In Figur 4 ist ein Konus 3 mit dem vorher erwähnten Feinstgewinde 18 gezeigt. Entsprechend dem vorgesehenen Abstand 27 zwischen dem Mittelpunkt einer Femurkugel und der Stirnfläche von dem befestigten Konus 3 wird der Abstand 27 zwischen Bechergrund und Mittelpunkt 11 der Probierkugel 1 gewählt, um den Einsatz 2 in der Probierkugel 1 zu verankern. Für den Tiefenanschlag des Einsatzes 2 muss der Abstand 27 um ein Bearbeitungsmass 28 vergrössert werden.

In Figur 3 geht der Hals 25 einer Raspel (nicht gezeigt) in einen Konus 3 über, auf den eine Probierkugel 1 mit Einsatz 2 aufgesetzt werden kann, um mit einer in den Femurknochen eingeschlagenen Raspel die Artikulation zu überprüfen. Der Konus 3 der Raspel ist mit einem Ausschnitt 14 versehen, der zur Kraftübertragung beim Ausschlagen der Raspel dient. Ausserdem ist der Konus 3 mit einer Rinne 15 versehen, in welche die Biegefedern 5 mit ihren Innen-Nocken einrasten, wenn die Stirnfläche 16 vom Konus 3 im Grund des Einsatzes 2 anschlägt.

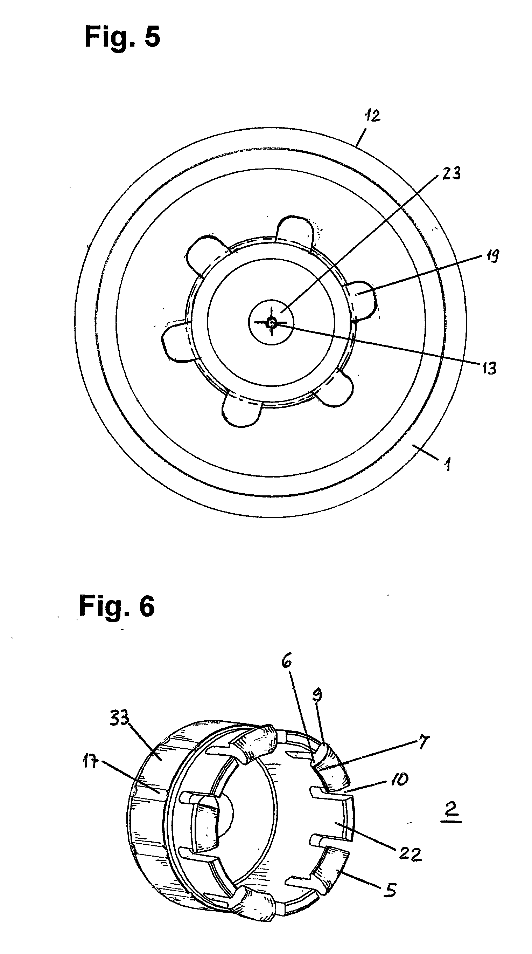

Der Einsatz 2 in Figur 6 besteht aus gespritztem Kunststoff aus der Gruppe der Polyetheretherketone (PEK, PEEK, PAEK, PEEKK etc.) und ist für Probierkugeln 1 mit unterschiedlichem Radius 21 und mit unterschiedlichem Abstand 27 vom Kugelmittelpunkt 11 zur Stirnfläche 16 eines gleichen Konus 3 vorgesehen. Man erkennt die Längsschlitze 10, welche den becherförmigen Einsatz 2 an seinem Rand in Stützlappen 22 und in Biegefedern 5 mit Innen-Nocken 6 und Aussen-Nocken 9 unterteilen. Auf der Aussenseite ist der zylindrische Vorsprung 33 für eine Schnappverbindung angebracht, der zur besseren Sterilisation von Kanälen 17 durchsetzt ist.

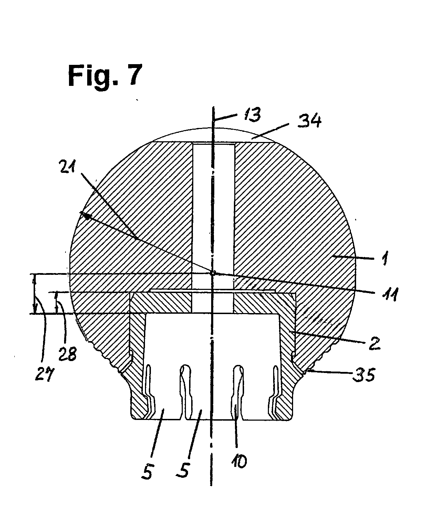

In Figur 7 ist ein weiteres Beispiel gezeigt, bei welchem die Probierkugel für eine Prothesenkugel verwendet wird, deren konische Gegenfläche 4 in einem über die Kugelfläche vorstehenden Hals endet. Der Einsatz 2 hat ebenfalls Becherform und ist durch Längsschlitze 10 in Biegefedern 5 unterteilt. Die Biegefedern 5 haben über die konische Gegenfläche 4 vorstehende Innen-Nocken 6, die beim Aufschieben auf einen Konus 3 eine Haltekraft erzeugen. Die Biegefedern 5 sind durch eine Abstützung 35 an der Probierkugel 1, die jeweils etwa in der Hälfte der Biegefedern 5 angreift, an einer weiteren radialen Auslenkung gehindert, um neben der Zentrierung im Bechergrund eine Zentrierung auf der Höhe der Innen-Nocken zu erreichen.

## Patentansprüche

1. Probierkugeln für Hüftgelenkprothesen, welche eine Konusverbindung zwischen einem Prothesenhals (24) mit Konus (3) und aufsetzbaren Hüftgelenkkugeln aufweisen, wobei die Hüftgelenkkugeln und die zugehörigen Probierkugeln (1) jeweils praktisch gleiche Radien (21) und jeweils die gleiche Lage ihrer konischen Gegenfläche (4) relativ zum Kugelmittelpunkt (11) aufweisen, **dadurch gekennzeichnet, dass** die Probierkugeln (1) einen separaten Einsatz (2) mit vorbestimmter Federwirkung für den Kontakt zum Konus (3) aufweisen, wobei der Einsatz in den Probierkugeln (1) in unterschiedlichem Abstand (27) längs der Polachse (13) zum Kugelmittelpunkt (11) befestigbar ist.

2. Probierkugeln nach Anspruch 1, **dadurch gekennzeichnet, dass** Probierkugeln (1) einer Konusgrösse einen gleichen separaten Einsatz (2) aufweisen.

3. Probierkugeln nach einem der Ansprüche 1 oder 2, **dadurch gekennzeichnet, dass** die Probierkugeln (1) und der Einsatz (2) aus Kunststoff bestehen.

4. Probierkugel nach einem der Ansprüche 1 bis 2, **dadurch gekennzeichnet, dass** der Einsatz als Spritzling durch ein Spritzwerkzeug herstellbar ist.

5. Probierkugel nach Anspruch 1, **dadurch gekennzeichnet, dass** der Einsatz (2) aus Metall besteht.

6. Probierkugel nach einem der vorangehenden Ansprüche, **dadurch gekennzeichnet, dass** der Einsatz (2) die Form eines Bechers hat, dessen Rand durch Längsschlitze (10) in die konische Gegenfläche (4) fortsetzende Stützlappen (22) und in Biegefedern (5) mit über die Konusform vorstehenden Innen-Nocken (6) unterteilt ist, um den Konus (3) mit den Stützlappen (22) zu führen und mit den durch die Biegefedern (5) erzeugten Radialkräften durch Reibung am Herausgleiten zu hindern.

7. Probierkugel nach Anspruch 6, **dadurch gekennzeichnet, dass** die Stützlappen (22) radial in der Probierkugel (1) abgestützt sind.

8. Probierkugel nach Anspruch 6 oder 7, **dadurch gekennzeichnet, dass** die Biegefedern (5) des Einsatzes (2) mit Aussen-Nocken (9) versehen sind, welche zur Drehsicherung des Einsatzes (2) in Längsnuten (19) an der Probierkugel (1) eingreifen.

9. Probierkugel nach einem der Ansprüche 1 bis 8, **dadurch gekennzeichnet, dass** der Einsatz (2) über eine Schnappverbindung (26) in der Probierkugel (1) gehalten ist.

10. Probierkugel nach einem der Ansprüche 6 bis 9, **dadurch gekennzeichnet, dass** die Innen-Nocken (6) auf einer Raspel mit Konus (3) in eine Rinne (15) oder einen Ausschnitt (14) eingreifen, um ein Herausgleiten der Probierkugel (1) zu verhindern.

11. Probierkugel nach einem der Ansprüche 1 bis 5, **dadurch gekennzeichnet, dass** der Einsatz (2) die Form eines Bechers hat, dessen Rand durch Längsschlitze (10) in Biegefedern (5) mit über die Konusform vorstehenden Innen-Nocken (6) unterteilt ist, um über eine dem Überstand der Innen-Nocken (6) entsprechende Auslenkung der Biegefedern (5) eine Haltekraft zu erzeugen, wobei die Biegefedern (5) an einer Fortsetzung der Auslenkung durch eine zusätzliche Abstützung in der Probierkugel (1) gehindert sind, um eine Zentrierung beim Aufsetzen auf einen Konus (3) zu erreichen.

12. Probierkugel nach einem der Ansprüche 1 bis 11, **dadurch gekennzeichnet, dass** die Kontaktflächen zwischen Einsatz (2) und Probierkugel (1) Kanäle (17) nach aussen aufweisen, um auch die Sterilisation in den Kontaktflächen sicherzustellen.
